# EUROPÄISCHE PATENTANMELDUNG

(11) **EP 0 834 551 A2**
(43) Veröffentlichungstag der Anmeldung: **08.04.1998**
(21) Anmeldenummer: 97116695.4
(22) Anmeldetag: 25.09.1997
(51) Int. Cl.: C11D 3/50, C11D 1/66, A61K 7/46

(54) **Fixierung von Duftstoffen aus Wasch- und Reinigungsmitteln an Oberflächen**

(30) Priorität: 04.10.1996 DE 19640986
(71) Anmelder: Henkel Kommanditgesellschaft auf Aktien, 40589 Düsseldorf-Holthausen (DE)
(72) Erfinder: Meine, Georg, Dipl.-Chem. Dr., 40822 Mettmann (DE); Schaper, Ulf-Armin, Dipl.-Chem. Dr., 47804 Krefeld (DE); Hofmann, Matthias, Dipl.-Physiker Dr., 40789 Monheim (DE); Ruhnke, Anja, 40764 Langenfeld (DE)

(57) **Zusammenfassung**

Die Haftung von Duftstoffen an Oberflächen, zum Beispiel von Textilien, harten Gegenständen oder des menschlichen Körpers, sollte verbessert werden. Dies gelang im wesentlichen durch die Verwendung von Alkylpolyglykosiden der allgemeinen Formel R¹-O(G)ₙ, in der R¹ einen Alkylrest mit 8 bis 22 C-Atomen, G eine Glykoseeinheit und n eine Zahl von 1 bis 10 bedeuten, zur Fixierung von Duftstoffen an harten und/oder weichen Oberflächen. Die Wasch- beziehungsweise Reinigungsleistung der Alkylpolyglykoside, insbesondere bei niedrigen Temperaturen, wird durch den Einsatz der Duftstoffe erhöht.

## Beschreibung

Die vorliegende Erfindung betrifft die Verwendung von Alkylpolyglykosiden zur Fixierung von Duftstoffen an harten und weichen Oberflächen, wie zum Beispiel an Textilien, insbesondere bei deren Wäsche, sowie Wasch- und Reinigungsmittel, welche eine Kombination aus Alkylglykosid und bestimmtem Duftstoff enthalten.

Bei der Wäsche von Textilien beziehungsweise der Reinigung harter Oberflächen wie zum Beispiel Badezimmerfliesen erwartet man nicht nur eine optisch einwandfreie Sauberkeit, sondern auch das Fehlen von etwaigen unangenehmen Gerüchen auf der gereinigten textilen beziehungsweise harten Oberfläche. Im Gegenteil wird oft ein Zurückbleiben von Duftstoffen, die aus dem Reinigungs-, Wasch- oder Wäschenachbehandlungsmittel stammen, als angenehm empfunden und verstärkt den Sauberkeitseindruck. Zum Beispiel beim manuellen Waschen von Textilien, das normalerweise im Waschbecken durchgeführt wird, wird von vielen Anwendern der zurückbleibende Geruch im Becken sowie an den Händen als angenehm empfunden.

Aus der internationalen Patentanmeldung WO 95/04809 ist ein Verfahren zum Parfümieren von Textilien beim Waschen mit lipasehaltigen Waschmitteln bekannt, wobei bestimmte estergruppenhaltige Duftstoffe eingesetzt werden. Die europäische Patentanmeldung EP 0 430 315 betrifft lipasehaltige Waschmittel, die Duftstoffe enthalten, wobei bestimmte Duftstofffkomponenten eine gewisse Gehaltsobergrenze nicht überschreiten sollen, während andere Duftstoffkomponenten eine gewisse Gehaltsuntergrenze nicht unterschreiten dürfen. Dadurch soll sowohl der Lipase-Eigengeruch als auch der Geruch der lipolytisch aus Fetten entstehenden Produkte überdeckt werden.

An Inhaltsstoffe moderner Wasch- und Reinigungsmittel werden üblicherweise aber höhere Anforderungen gestellt als daß sie nur das subjektive Empfinden des Anwenders beeinflussen sollen. So sollen möglichst alle Bestandteile des Mittels zum Waschbeziehungsweise Reinigungsergebnis beitragen.

Übertaschenderweise wurde nun gefunden, daß durch den Einsatz bestimmter nichtionischer Tenside die Haftung von Duftstoffen an Oberflächen, zum Beispiel von Textilien, harten Gegenständen oder des menschlichen Körpers, verbessert werden kann, wenn man sie zusammen beim Waschen beziehungsweise Reinigen verwendet. Umgekehrt wird überraschenderweise die Wasch- beziehungsweise Reinigungsleistung der bestimmten Tenside, insbesondere bei niedrigen Temperaturen, durch den Einsatz der Duftstoffe erhöht.

Gegenstand der Erfindung ist die Verwendung von Alkylpolyglykosiden der allgemeinen Formel I

R¹-O(G)ₙ (I)

in der R¹ einen Alkylrest mit 8 bis 22 C-Atomen, G eine Glykoseeinheit und n eine Zahl von 1 bis 10 bedeuten, zur Fixierung von Duftstoffen an harten und/oder weichen Oberflächen. Unter weichen Oberflächen sollen in diesem Zusammnenhang sowohl menschliche Haut als auch Haar und Textilien unterschiedlicher Zusammensetzung, zum Beispiel aus Baumwolle, Wolle, Seide, Polyester und Mischgeweben jeglicher Art, verstanden werden. Vorzugsweise wird das Alkylpolyglykosid in Gewichtsmengen, bezogen auf den Duftstoff, im Bereich von 20:1 bis 1:1, insbesondere von 5:1 bis 1:1, verwendet.

Ein weiterer Gegenstand der Erfindung ist die Verwendung von Duftstoffen zur Erhöhung der Wasch- und/oder Reinigungsleistung von Alkylpolyglykosiden der allgemeinen Formel I

R¹-O(G)ₙ (I)

in der R¹ einen Alkylrest mit 8 bis 22 C-Atomen, G eine Glykoseeinheit und n eine Zahl von 1 bis 10 bedeuten.

Als geeignete Duftstoffe können einzelne Riechstoffverbindungen, z.B. die synthetischen Produkte vom Typ der Ester, Ether, Aldehyde, Ketone, Alkohole und Kohlenwasserstoffe verwerden werden. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 - 18 C-Atomen, Citral, Citronellal, Citronellyloxyacetaldehyd, Cyclamenaldehyd, Hydroxycitronellal, Lilial und Bourgeonal, zu den Ketonen z.B. die Jonone, ∝-Isomethylionon und Methyl-cedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen.

Solche Parfümöle können auch natürliche Riechstoffgemische enthalten, wie sie aus pflanzlichen oder tierischen Quellen zugänglich sind, z.B. Pinien-, Citrus-, Jasmin-, Lilien-, Rosen- oder Ylang-Ylang-Öl. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wachol-derbeeröl, Vetiveröl, Olibanumöl, Galbanumöl, Labdanumöl und Lavandinöl.

Vorzugsweise werden Bergamotteöl, Dihydromyrcenol, Lilial, Lyral, Citronellol, Phenylethylalkohol, ∝-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

Noch ein weiterer Gegenstand der Erfindung ist ein Verfahren zur Fixierung von Duftstoffen an harten und/oder weichen Oberflächen, insbesondere an Textilien, welches dadurch gekennzeichnet ist, daß man die Oberfläche mit dem Duftstoff und einem Alkylpolyglykosid der allgemeinen Formel I

R¹-O(G)ₙ (I)

in der R¹ einen Alkylrest mit 8 bis 22 C-Atomen, G eine Glykoseeinheit und n eine Zahl von 1 bis 10 bedeuten, insbesondere unter Einwirkung von mechanischen Kräften wie sie in haushaltsüblichen Waschmaschinen auftreten, in Gegenwart von Wasser über einen Zeitraum von 2 Minuten bis 90 Minuten bei einer Temperatur unter 95 °C, insbesondere im Bereich von 20 °C bis 60 °C behandelt. Bevorzugt sind Behandlungsdauern im Bereich von 5 Minuten bis 60 Minuten, insbesondere von 15 Minuten bis 45 Minuten. Die Temperatur liegt vorzugsweise im Bereich von 20 °C bis 60 °C, insbesondere von 20 °C bis 40 °C, wobei besonders bevorzugt während der gesamten Behandlungszeit der Oberfläche mit der erfindungsgemäß verwendeten Kombination die Temperatur im Bereich von 20 °C bis 40 °C liegt. Die Konzentration des Alkylpolyglykosids der allgemeinen Formel I liegt beim erfindungsgemäßen Verfahren vorzugsweise im Bereich von 0,05 g/l bis 5 g/l, insbesondere von 0,1 g/l bis 3 g/l, während die Konzentration an Duftstoff in der wäßrigen Behandlungsflotte vorzugsweise im Bereich von 0,004 g/l bis 0,12 g/, insbesondere von 0,02 g/l bis 0,04 g/l liegt.

Besonders vorteilhaft kann das erfindungsgemäße Verfahren mit Hilfe eines Wasch- beziehungsweise Reinigungsmittels durchgeführt werden, welches einen oben definierten geeigneten Duftstoff und das Alkylglykosid enthält. Ein weiterer Gegenstand der Erfindung ist daher ein Wasch- oder Reinigungsmittel, enthaltend einen derartigen Duftstoff und ein Alkylpolyglykosid der allgemeinen Formel I

R¹-O(G)ₙ (I)

in der R¹ einen Alkylrest mit 8 bis 22 C-Atomen, G eine Glykoseeinheit und n eine Zahl von 1 bis 10 bedeuten. In erfindungsgemäßen Mitteln sind vorzugsweise 1 Gew.-% bis 50 Gew.-%, insbesondere 1 Gew.-% bis 10 Gew.-% derartiger Duftstoffe vorhanden. Ein erfindungsgemäßes Mittel kann als solches oder nach Verdünnen mit Wasser im erfindungsgemäßen Verfahren beziehungsweise im Rahmen der erfindungsgemäßen Verwendung eingesetzt werden.

Bei einem derartigen Mittel kann es sich insbesondere um ein Textilwaschmittel, welches in teilchenförmiger oder flüssiger Form vorliegen kann, ein in entsprechender Form vorliegendes Reinigungsmittel für harte Oberflächen, zum Beispiel einen Bad- oder Sanitärreiniger, oder ein Reinigungsmittel für den menschlichen Körper, zum Beispiel ein Haarshampoo, eine Reinigungslotion oder eine Stückseife, handeln. Insbesondere ist die erfindungsgemäße Lehre im Körperpflegebereich aber einsetzbar für Dauerwellpräparate und Depilatorien sowie für Mittel, die, z.B. zur Einstellung des pH-Wertes, niedere Amine enthalten.

Im Rahmen der Erfindung geeignete Alkylglykoside und ihre Herstellung werden zum Beispiel in den europäischen Patentanmeldungen EP 0 092 355, EP 0 301 298, EP 0 357 969 und EP 0 362 671 oder der US-amerikanischen Patentschrift US 3 547 828 beschrieben. Bei den Glykosidkomponenten (Gₙ in Formel I) derartiger Alkylglykoside handelt es sich um Oligo- oder Polymere aus natürlich vorkommenden Aldose- oder Ketose-Monomeren, zu denen insbesondere Glucose, Mannose, Fruktose, Galaktose, Talose, Gulose, Altrose, Allose, Idose, Ribose, Arabinose, Xylose und Lyxose gehören. Die aus derartigen glykosidisch verknüpften Monomeren bestehenden Oligomere werden außer durch die Art der in ihnen enthaltenen Zucker durch deren Anzahl, den sogenannten Oligomerisierungsgrad, charakterisiert. Der Oligomerisierungsgrad (n in Formel I) kann als analytisch zu ermittelnde Größe auch gebrochene Zahlenwerte annehmen; er liegt in der Regel bei Werten zwischen 1 und 10, bei den vorzugsweise eingesetzten Alkylglykosiden unter einem Wert von 1,5, insbesondere zwischen 1,2 und 1,4. Bevorzugter Monomer-Baustein ist wegen der guten Verfügbarkeit Glucose. Der Alkylteil (R¹ in Formel I) der erfindungsgemäß verwendeten Alkylglykoside stammt bevorzugt ebenfalls aus leicht zugänglichen Derivaten nachwachsender Rohstoffe, insbesondere aus Fettalkoholen, obwohl auch deren verzweigtkettige Isomere, insbesondere sogenannte Oxoalkohole, zur Herstellung verwendbarer Alkylglykoside eingesetzt werden können. Brauchbar sind demgemäß insbesondere die primären Alkohole mit linearen Octyl-, Decyl-, Dodecyl-, Tetradecyl-, Hexadecyl- oder Octadecylresten sowie deren Gemische. Besonders geeignete Alkylglykoside enthalten einen Kokosfettalkylrest, das heißt Mischungen mit im wesentlichen R¹=Dodecyl und R¹=Tetradecyl. Die Alkylglykoside können herstellungsbedingt geringe Mengen, beispielsweise 1 bis 2 % bezogen auf Alkylglykosid, an nicht umgesetztem freiem Fettalkohol enthalten, was sich in der Regel nicht nachteilig auf ihre Verwendung und die Eigenschaften der daraus hergestellten Mittel auswirkt. Alkylglykoside sind in erfindungsgemäßen Mitteln vorzugsweise in Mengen von 0,5 Gew.-% bis 15 Gew.-%, insbesondere von 1 Gew.-% bis 5 Gew.-% enthalten. Das Gewichtsverhältnis von Alkylglykosid zu Duftstoff beträgt in erfindungsgemäßen Mitteln vorzugsweise 10:1 bis 1:2, insbesondere 2:1 bis 1:1.

Besonders ausgeprägt zeigt sich die reinigungsverstärkende Wirkung der Duftstoffe bei Verschmutzungen auf Mineralölbasis, die von Lipasen naturgemäß nicht hydrolytisch angegriffen werden können. In einer bevorzugten Ausgestaltung der Erfindung wird der Duftstoff daher in Gegenwart einer Lipase verwndet und ist ein erfindungsgemäßes Mittel lipasehaltig, so daß sowohl mineralölbasierte als auch glyzerinesterbasierte fetthaltige Anschmutzungen, zum Beispiel an Textilien, gut entfernt werden. Vorzugsweise enthält ein erfindungsgemäßes Mittel Lipase in einer solchen Menge, daß es eine lipolytische Aktivität im Bereich von 2 LU bis 2000 LU, insbesondere von 2 LU bis 100 LU pro Gramm des Mittels aufweist.

Zusätzlich können erfindungsgemäße beziehungsweise im erfindungsgemäßen Verfahren eingesetzte Mittel, die als insbesondere pulverförmige Feststoffe, in nachverdichteter Teilchenform, als homogene Lösungen oder Suspensionen vorliegen können, alle üblichen Inhaltsstoffe aufweisen, solange sie nicht mit dem Alkylglykosid oder insbesondere dem Dufststoff so in Wechselwirkung treten, daß der erwünschte Effekt der Verstärkung der Duftfixierung an der Oberfläche ausbleibt.

Erfindungsgemäße Wasch- oder Reirigumgsmittel können insbesondere Buildersubstanzen, zusätzlich zum Alkylpolyglykosid weitere oberflächenaktive Tenside, Enzyme, organische und/oder anorganische Persauerstoffverbindungen, Persauerstoff-Aktivatoren, wassermischbare organische Lösungsmittel, Sequestrierungsmittel, Elektrolyte, pH-Regulatoren und weitere Hilfsstoffe, wie soil release-Wirkstoffe, optische Aufheller, Vergrauungsinhibitoren, Farbübertragungsinhibitoren, Schaumregulatoren sowie Farbstoffe enthalten.

Die erfindungsgemäßen Mittel können neben dem Alkylglykosid weitere Tenside enthalten, wobei insbesondere anionische Tenside, nichtionische Tenside und deren Gemische in Frage kommen. Geeignete nichtionische Tenside sind insbesondere Ethoxylierungs- und/oder Propoxylierungsprodukte von Alkylglykosiden und/oder linearen oder verzweigten Alkoholen mit jeweils 12 bis 18 C-Atomen im Alkylteil und 3 bis 20, vorzugsweise 4 bis 10 Alkylethergruppen. Weiterhin sind entsprechende Ethoxylierungs- und/oder Propoxylierungsprodukte von N-Alkylaminen, vicinalen Diolen, Fettsäureestern und Fettsäureamiden, die hinsichtlich des Alkylteils den genannten langkettigen Alkoholderivaten entsprechen, sowie von Alkylphenolen mit 5 bis 12 C-Atomen im Alkylrest brauchbar.

Geeignete anionische Tenside sind insbesondere Seifen und solche, die Sulfat- oder Sulfonat-Gruppen mit bevorzugt Alkaliionen als Kationen enthalten. Verwendbare Seifen sind bevorzugt die Alkalisalze der gesättigten oder ungesättigten Fettsäuren mit 12 bis 18 C-Atomen. Derartige Fettsäuren können auch in nicht vollständig neutralisierter Form eingesetzt werden. Zu den brauchbaren Tensiden des Sulfat-Typs gehören die Salze der Schwefelsäurehalbester von Fettalkoholen mit 12 bis 18 C-Atomen und die Sulfatierungsprodukte der genannten nichtionischen Tenside mit niedrigem Ethoxylierungsgrad. Zu den verwendbaren Tensiden vom Sulfonat-Typ gehören lineare Alkylbenzolsulfonate mit 9 bis 14 C-Atomen im Alkylteil, Alkansulfonate mit 12 bis 18 C-Atomen, sowie Olefinsulfonate mit 12 bis 18 C-Atomen, die bei der Umsetzung entsprechender Monoolefine mit Schwefeltrioxid entstehen, sowie alpha-Sulfofettsäureester, die bei der Sulfonierung von Fettsäuremethyl- oder -ethylestern entstehen.

Derartige zusätzliche Tenside sind in den erfindungsgemäßen Waschmitteln in Mengenanteilen von vorzugsweise 5 Gew.-% bis 50 Gew.-%, insbesondere von 8 Gew.-% bis 30 Gew.-%, enthalten.

Ein erfindungsgemäßes Mittel enthält vorzugsweise mindestens einen wasserlöslichen und/oder wasserunlöslichen, organischen und/oder anorganischen Builder. Zu den wasserlöslichen organischen Buildersubstanzen gehören Polycarbonsäuren, insbesondere Citronensäure und Zuckersäuren, monomere und polymere Aminopolycarbonsäuren, insbesondere Methylglycindiessigsäure, Nitrilotriessigsäure und Ethylendiamintetraessigsäure sowie Polyasparaginsäure, Polyphosphonsäuren, insbesondere Aminotris(methylenphosphonsäure), Ethylendiamintetrakis(methylenphosphonsäure) und 1-Hydroxyethan-1,1-diphosphonsäure, polymere Hydroxyverbindungen wie Dextrin sowie polymere (Poly-)carbonsäuren, insbesondere die durch Oxidation von Polysacchariden zugänglichen Polycarboxylate der europäischen Patentschrift EP 0 609 273, polymere Acrylsäuren, Methacrylsäuren, Maleinsäuren und Mischpolymere aus diesen, die auch geringe Anteile polymerisierbarer Substanzen ohne Carbonsäurefunktionalität einpolymerisiert enthalten können. Die relative Molekülmasse der Homopolymeren ungesättiger Carbonsäuren liegt im allgemeinen zwischen 5 000 und 200 000, die der Copolymeren zwischen 2 000 und 200 000, vorzugsweise 50 000 bis 120 000, jeweils bezogen auf freie Säure. Ein besonders bevorzugtes Acrylsäure-Maleinsäure-Copolymer weist eine relative Molekülmasse von 50 000 bis 100 000 auf. Geeignete, wenn auch weniger bevorzugte Verbindungen dieser Klasse sind Copolymere der Acrylsäure oder Methacrylsäure mit Vinylethern, wie Vinylmethylethern, Vinylester, Ethylen, Propylen und Styrol, in denen der Anteil der Säure mindestens 50 Gew.-% beträgt. Als wasserlösliche organische Buildersubstanzen können auch Terpolymere eingesetzt werden, die als Monomere zwei ungesättigte Säuren und/oder deren Salze sowie als drittes Monomer Vinylalkohol und/oder ein Vinylalkohol-Derivat oder ein Kohlenhydrat enthalten. Das erste saure Monomer beziehungsweise dessen Salz leitet sich von einer monoethylenlsch ungesättigten C₃-C₈-Carbonsäure und vorzugsweise von einer C₃-C₄-Monocarbonsäure, insbesondere von (Meth)-acrylsäure ab. Das zweite saure Monomer beziehungsweise dessen Salz kann ein Derivat einer C₄-C₈-Dicarbonsäure sein, wobei Maleinsäure besonders bevorzugt ist. Die dritte monomere Einheit wird in diesem Fall von Vinylalkohol und/oder vorzugsweise einem veresterten Vinylalkohol gebildet. Insbesondere sind Vinylalkohol-Derivate bevorzugt, welche einen Ester aus kurzkettigen Carbonsäuren, beispielsweise von C₁-C₄-Carbonsäuren, mit Vinylalkohol darstellen. Bevorzugte Polymere enthalten dabei 60 Gew.-% bis 95 Gew.-%, insbesondere 70 Gew.-% bis 90 Gew.-% (Meth)acrylsäure bzw. (Meth)acrylat, besonders bevorzugt Acrylsäure bzw. Acrylat, und Maleinsäure bzw. Maleinat sowie 5 Gew.-% bis 40 Gew.-%, vorzugsweise 10 Gew.-% bis 30 Gew.-% Vinylalkohol und/oder Vinylacetat. Ganz besonders bevorzugt sind dabei Polymere, in denen das Gewichtsverhältnis von (Meth)acrylsäure beziehungsweise (Meth)acrylat zu Maleinsäure beziehungsweise Maleinat zwischen 1:1 und 4:1, vorzugsweise zwischen 2:1 und 3:1 und insbesondere 2:1 und 2,5:1 liegt. Dabei sind sowohl die Mengen als auch die Gewichtsverhältnlsse auf die Säuren bezogen. Das zweite saure Monomer beziehungsweise dessen Salz kann auch ein Derivat einer Allylsulfonsäure sein, die in 2-Stellung mit einem Alkylrest, vorzugsweise mit einem C₁-C₄-Alkylrest, oder einem aromatischen Rest, der sich vorzugsweise von Benzol oder Benzol-Derivaten ableitet, substituiert ist. Bevorzugte Terpolymere enthalten dabei 40 Gew.-% bis 60 Gew.-%, insbesondere 45 bis 55 Gew.-% (Meth)acrylsäure beziehungsweise (Meth)acrylat, besonders bevorzugt Acrylsäure beziehungsweise Acrylat, 10 Gew.-% bis 30 Gew.-%, vorzugsweise 15 Gew.-% bis 25 Gew.-% Methallylsulfonsäure bzw. Methallylsulfonat und als drittes Monomer 15 Gew.-% bis 40 Gew.-%, vorzugsweise 20 Gew.-% bis 40 Gew.-% eines Kohlenhydrats. Dieses Kohlenhydrat kann dabei beispielsweise ein Mono-, Di-, Oligo- oder Polysaccharid sein, wobei Mono-, Di- oder Oligosaccharide bevorzugt sind. Besonders bevorzugt ist Saccharose. Durch den Einsatz des dritten Monomers werden vermutlich Sollbruchstellen in das Polymer eingebaut, die für die gute biologische Abbaubarkeit des Polymers verantwortlich sind. Diese Terpolymere lassen sich insbesondere nach Verfahren herstellen, die in der deutschen Patentschrift DE 42 21 381 und der deutschen Patentanmeldung DE 43 00 772 beschrieben sind, und weisen im allgemeinen eine relative Molekülmasse zwischen 1 000 und 200 000, vorzugsweise zwischen 200 und 50 000 und insbesondere zwischen 3 000 und 10 000 auf. Weitere bevorzugte Copolymere sind solche, die in den deutschen Patentanmeldungen DE 43 03 320 und DE 44 17 734 beschrieben werden und als Monomere vorzugsweise Acrolein und Acrylsäure/Acrylsäuresalze beziehungsweise Vinylacetat aufweisen. Die organischen Buildersubstanzen können, insbesondere zur Herstellung flüssiger Mittel in Form wäßriger Lösungen, vorzugsweise in Form 30- bis 50-gewichtsprozentiger wäßriger Lösungen eingesetzt werden. Alle genannten Säuren werden in der Regel in Form ihrer wasserlöslichen Salze, insbesondere ihre Alkalisalze, eingesetzt.

Derartige organische Buildersubstanzen können gewünschtenfalls in Mengen bis zu 40 Gew.-%, insbesondere bis zu 25 Gew.-% und vorzugsweise von 1 Gew.-% bis 8 Gew.-% enthalten sein. Mengen nahe der genannten Obergrenze werden vorzugsweise in pastenförmigen oder flüssigen, insbesondere wasserhaltigen, erfindungsgemäßen Mitteln eingesetzt.

Als wasserlösliche anorganische Buildermaterialien kommen insbesondere Alkalisilikate und Polyphosphate, vorzugsweise Natriumtriphosphat, in Betracht. Als wasserunlösliche, wasserdispergierbare anorganische Buildermaterialien werden insbesondere kristalline oder amorphe Alkalialumosilikate, in Mengen von bis zu 50 Gew.-%, vorzugsweise nicht über 40 Gew.-% und in flüssigen Mitteln insbesondere von 1 Gew.-% bis 5 Gew.-%, eingesetzt. Unter diesen sind die kristallinen Natriumalumosilikate in Waschmittelqualität, insbesondere Zeolith A, P und gegebenenfalls X, bevorzugt. Mengen nahe der genannten Obergrenze werden vorzugsweise in festen, teilchenförmigen Mitteln eingesetzt. Geeignete Alumosilikate weisen insbesondere keine Teilchen mit einer Korngröße über 30 µm auf und bestehen vorzugsweise zu wenigstens 80 Gew.-% aus Teilchen mit einer Größe unter 10 µm. Ihr Calciumbindevermögen, das nach den Angaben der deutschen Patentschrift DE 24 12 837 bestimmt werden kann, liegt in der Regel im Bereich von 100 bis 200 mg CaO pro Gramm.

Geeignete Substitute beziehungsweise Teilsubstitute für das genannte Alumosilikat sind kristalline Alkalisilikate, die allein oder im Gemisch mit amorphen Silikaten vorliegen können. Die in den erfindungsgemäßen Mitteln als Gerüststoffe brauchbaren Alkalisilikate weisen vorzugsweise ein molares Verhältnis von Alkalioxid zu SiO₂ unter 0,95, insbesondere von 1:1,1 bis 1:12 auf und können amorph oder kristallin vorliegen. Bevorzugte Alkalisilikate sind die Natriumsilikate, insbesondere die amorphen Natriumsilikate, mit einem molaren Verhältnis Na₂O:SiO₂ von 1:2 bis 1:2,8. Solche mit einem molaren Verhältnis Na₂O:SiO₂ von 1:1,9 bis 1:2,8 können nach dem Verfahren der europäischen Patentanmeldung EP 0 425 427 hergestellt werden. Als kristalline Silikate, die allein oder im Gemisch mit amorphen Silikaten vorliegen können, werden vorzugsweise kristalline Schichtsilikate der allgemeinen Formel Na₂SiₓO₂ₓ₋₁ · y H₂O eingesetzt, in der x, das sogenannte Modul, eine Zahl von 1,9 bis 4 und y eine Zahl von 0 bis 20 ist und bevorzugte Werte für x 2, 3 oder 4 sind. Kristalline Schichtsilikate, die unter diese allgemeine Formel fallen, werden beispielsweise in der europäischen Patentanmeldung EP 0 164 514 beschrieben. Bevorzugte kristalline Schichtsilikate sind solche, bei denen x in der genannten allgemeinen Formel die Werte 2 oder 3 annimmt. Insbesondere sind sowohl ß- als auch δ-Natriumdisilikate (Na₂Si₂O₅ · y H₂O) bevorzugt, wobei ß-Natriumdisilikat beispielsweise nach dem Verfahren erhalten werden kann, das in der internationalen Patentanmeldung WO 91/08171 beschrieben ist. δ-Natriumsilikate mit einem Modul zwischen 1,9 und 3,2 können gemäß den japanischen Patentanmeldungen JP 04/238 809 oder JP 04/260 610 hergestellt werden. Auch aus amorphen Alkalisilikaten hergestellte, praktisch wasserfreie kristalline Alkalisilikate der obengenannten allgemeinen Formel, in der x eine Zahl von 1,9 bis 2,1 bedeutet, herstellbar wie in den europäischen Patentanmeldungen EP 0 548 599, EP 0 502 325 und EP 0 452 428 beschrieben, können in erfindungsgemäßen Mitteln eingesetzt werden. In einer weiteren bevorzugten Ausführungsform erfindungsgemäßer Mittel wird ein kristallines Natriumschichtsilikat mit einem Modul von 2 bis 3 eingesetzt, wie es nach dem Verfahren der europäischen Patentanmeldung EP 0 436 835 aus Sand und Soda hergestellt werden kann. Kristalline Natriumsilikate mit einem Modul im Bereich von 1,9 bis 3,5, wie sie nach den Verfahren der europäischen Patentschriften EP 0 164 552 und/oder EP 0 293 753 erhältlich sind, werden in einer weiteren bevorzugten Ausführungsform erfindungsgemäßer Mittel eingesetzt. Falls als zusätzliche Buildersubstanz auch Alkalialumosilikat, insbesondere Zeolith, vorhanden ist, beträgt das Gewichtsverhältnis Alumosilikat zu Silikat, jeweils bezogen auf wasserfreie Aktivsubstanzen, vorzugsweise 1:10 bis 10:1. In Mitteln, die sowohl amorphe als auch kristalline Alkalisilikate enthalten, beträgt das Gewichtsverhältnis von amorphem Alkalisilikat zu kristallinem Alkalisilikat vorzugsweise 1:2 bis 2:1 und insbesondere 1:1 bis 2:1.

Buildersubstanzen sind in den erfindungsgemäßen Waschmitteln vorzugsweise in Mengen bis zu 60 Gew.-%, insbesondere von 5 Gew.-% bis 40 Gew.-%, enthalten.

Als geeignete Persauerstoffverbindungen kommen insbesondere organische Persäuren beziehungsweise persaure Salze organischer Säuren, wie Phthalimidopercapronsäure, Perberzoesäure oder Salze der Diperdodecandisäure, Wasserstoffperoxid und unter den Anwendungsbedingungen Wasserstoffperoxid abgebende anorganische Salze, wie Perborat, Percarbonat und/oder Persilikat, in Betracht. Sofern feste Perverbindungen eingesetzt werden sollen, können diese in Form von Pulvern oder Granulaten verwendet werden, die auch in im Prinzip bekannter Weise umhüllt sein können. Besonders bevorzugt wird Alkalipercarbonat, Alkaliperborat-Monohydrat oder insbesondere in flüssigen Mitteln Wasserstoffperoxid in Form wäßriger Lösungen, die 3 Gew.-% bis 10 Gew.-% Wasserstoffperoxid enthalten, eingesetzt. Falls ein erfindungsgemäßes Waschmittel Persauerstoffverbindungen enthält, sind diese in Mengen von vorzugsweise bis zu 50 Gew.-%, insbesondere von 5 Gew.-% bis 30 Gew.-%, vorhanden. Der Zusatz geringer Mengen bekannter Bleichmittelstabilisatoren wie beispielsweise von Phosphonaten, Boraten beziehungsweise Metaboraten und Metasilikaten sowie Magnesiumsalzen wie Magnesiumsulfat kann zweckdienlich sein.

Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diaceryl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat, 2,5-Diacetoxy-2,5-dihydrofuran und die aus den deutschen Patentanmeldungen DE 196 16 693 und DE 196 16 767 bekannten Enolester sowie acetyliertes Sorbitol und Mannitol beziehungsweise deren in der europäischen Patentanmeldung EP 0 525 239 beschriebene Mischungen (SORMAN), acylierte Zuckerderivate, insbesondere Pentaacetylglukose (PAG), Pentaacetylfruktose, Tetraacetylxylose und Octaacetyllactose sowie acetyliertes, gegebenenfalls N-alkyliertes Glucamin und Gluconolacton, und/oder N-acylierte Lactame, beispielsweise N-Benzoylcaprolactam, die aus den internationalen Patentanmeldungen WO 94/27970, WO 94/28102, WO 94/28103, WO 95/00626, WO 95/14759 und WO 95/17498 bekannt sind. Die aus der deutschen Patentanmeldung DE 196 16 769 bekannten hydrophil substituierten Acylacetale und die in der deutschen Patentanmeldung DE 196 16 770 sowie der internationalen Patentanmeldung WO 95/14075 beschriebenen Acyllactame werden ebenfalls bevorzugt eingesetzt. Auch die aus der deutschen Patentanmeldung DE 44 43 177 bekannten Kombinationen kon-ventioneller Bleichaktivatoren können eingesetzt werden. Derartige Bleichaktivatoren sind im üblichen Mengenbereich, vorzugsweise in Mengen von 1 Gew.-% bis 10 Gew.-%, insbesondere 2 Gew.-% bis 8 Gew.-%, bezogen auf gesamtes Mittel, enthalten.

Zusätzlich zu den oben aufgeführten konventionellen Bleichaktivatoren oder an deren Stelle können auch die aus den europäischen Patentschriften EP 0 446 982 und EP 0 453 003 bekannten Sulfonimine und/oder bleichverstärkende Übergangsmetallsalze beziehungsweise Übergangsmetallkomplexe als sogenannte Bleichkatalysatoren enthalten sein. Zu den in Frage kommenden Übergangsmetallverbindungen gehören insbesondere die aus der deutschen Patentanmeldung DE 195 29 905 bekannten Mangan-, Eisen-, Cobalt-, Ruthenium- oder Molybdän-Salenkomplexe und deren aus der deutschen Patentanmeldung DE 196 20 267 bekannte N-Analogverbindungen, die aus der deutschen Patentanmeldung DE 195 36 082 bekannten Mangan-, Eisen-, Cobalt-, Ruthenium- oder Molybdän-Carbonylkomplexe, die in der deutschen Patentanmeldung DE 196 05 688 beschriebenen Mangan-, Eisen-, Cobalt-, Ruthenium-, Molybdän-, Titan-, -Vanadium- und Kupfer-Komplexe mit stickstoffhaltigen Tripod-Liganden, die aus der deutschen Patentanmeldung DE 196 20 411 bekannten Cobalt-, Eisen-, Kupfer- und Ruthenium-Amminkomplexe, die in der deutschen Patentanmeldung DE 44 16 438 beschriebenen Mangan-, Kupfer- und Cobalt-Komplexe, die in der europäischen Patentanmeldung EP 0 272 030 beschriebenen Cobalt-Komplexe, die aus der europäischen Patentanmeldung EP 0 693 550 bekannten Mangan-Komplexe, die aus der europäischen Patentschrift EP 0 392 592 bekannten Mangan-, Eisen-, Cobalt- und Kupfer-Komplexe und/oder die in der europäischen Patentschrift EP 0 443 651 oder den europäischen Patentanmeldungen EP 0 458 397, EP 0 458 398, EP 0 549 271, EP 0 549 272, EP 0 544 490 und EP 0 544 519 beschriebenen Mangan-Komplexe. Kombinationen aus Bleichaktivatoren und Übergangsmetall-Bleichkatalysatoren sind beispielsweise aus der deutschen Patentanmeldung DE 196 13 103 und der internationalen Patentanmeldung WO 95/27775 bekannt. Bleichverstärkende Übergangsmetallkomplexe, insbesondere mit den Zentralatomen Mn, Fe, Co, Cu, Mo, V, Ti und/oder Ru, werden in üblichen Mengen, vorzugsweise in einer Menge bis zu 1 Gew.-%, insbesondere von 0,0025 Gew.-% bis 0,25 Gew.-% und besonders bevorzugt von 0,01 Gew.-% bis 0,1 Gew.-%, jeweils bezogen auf gesamtes Mittel, eingesetzt.

Als in den Mitteln verwendbare Erzyme kommen anstatt oder neben der oben erwähnten Lipase solche aus der Klasse der Proteasen, Cutinasen, Amylasen, Pullulanasen, Hemicellulasen, Cellulasen, Oxidasen und Peroxidasen sowie deren Gemische in Frage. Besonders geeignet sind aus Pilzen oder Bakterien, wie Bacillus subtilis, Bacillus licheniformis, Streptomyces griseus, Humicola lanuginosa, Humicola insolens, Pseudomonas pseudoalcaligenes oder Pseudomonas cepacia gewonnene enzymatische Wirkstoffe. Die gegebenenfalls verwendeten Enzyme können, wie zum Beispiel in den internationalen Patentanmeldungen WO 92/11347 oder WO 94/23005 beschrieben, an Trägerstoffen adsorbiert und/oder in Hüllsubstanzen eingebettet sein, um sie gegen vorzeitige Inaktivierung zu schützen. Sie sind in den erfindungsgemäßen Waschmitteln vorzugsweise nicht über 5 Gew.-%, insbesondere von 0,2 Gew.-% bis 2 Gew.-%, enthalten.

Die Mittel können als optische Aufheller beispielsweise Derivate der Diaminostilbendisulfonsäure beziehungsweise deren Alkalimetallsalze enthalten. Geeignet sind zum Beispiel Salze der 4,4'-Bis(2-anilino-4-morpholino-1,3,5-triazinyl-6-amino)stilben-2,2'-disulfonsäure oder gleichartig aufgebaute Verbindungen, die anstelle der Morpholino-Gruppe eine Diethanolaminogruppe, eine Methylaminogruppe, eine Anilinogruppe oder eine 2-Methoxyethylaminogruppe tragen. Weiterhin können Aufheller vom Typ der substituierten Diphenylstyryle anwesend sein, zum Beispiel die Alkalisalze des 4,4'-Bis(2-sulfostyryl)-diphenyls, 4,4'-Bis(4-chlor-3-sulfostyryl)-diphenyls oder 4-(4-Chlorstyryl)-4'-(2-sulfostyryl)-diphenyls. Auch Gemische der vorgenannten Aufheller können verwendet werden.

Zu den geeigneten Schauminhibitoren gehören beispielsweise Organopolysiloxane und deren Gemische mit mikrofeiner, gegebenenfalls silanierter Kieselsäure sowie Paraffinwachse und deren Gemische mit silanierter Kieselsäure oder Bisfettsäurealkylendiamiden. Mit Vorteilen werden auch Gemische aus verschiedenen Schauminhibitoren verwendet, zum Beispiel solche aus Silikonen, Paraffinen oder Wachsen. Vorzugsweise sind die Schauminhibitoren, insbesondere Silikon- und/oder Paraffinhaltige Schauminhibitoren, an eine granulare, in Wasser lösliche beziehungsweise dispergierbare Trägersubstanz gebunden. Insbesondere sind dabei Mischungen aus Paraffinwachsen und Bistearylethylendiamiden bevorzugt.

Zusätzlich können die Mittel auch Komponenten enthalten, welche die Öl- und Fettauswaschbarkeit aus Textilien positiv beeinflussen, sogenannte soil release-Wirkstoffe. Dieser Effekt wird besonders deutlich, wenn ein Textil verschmutzt wird, das bereits vorher mehrfach mit einem erfindungsgemäßen Waschmittel, das diese öl- und fettlösende Komponente enthält, gewaschen wurde. Zu den bevorzugten öl- und fettlösenden Komponenten zählen beispielsweise nicht-ionische Celluloseether wie Methylcellulose und Methylhydroxypropylcellulose mit einem Anteil an Methoxyl-Gruppen von 15 bis 30 Gew.-% und an Hydroxypropoxyl-Gruppen von 1 bis 15 Gew.-%, jeweils bezogen auf den nichtionischen Celluloseether, sowie die aus dem Stand der Technik bekannten Polymere der Phthalsäure und/oder der Terephthalsäure bzw. von deren Derivaten mit monomeren und/oder polymeren Diolen, insbesondere Polymere aus Ethylenterephthalaten und/oder Polyethylenglykolterephthalaten oder anionisch und/oder nichtionisch modifizierten Derivaten von diesen.

Zu den in den erfindungsgemäßen Mitteln, insbesondere wenn sie in flüssiger oder pastöser Form vorliegen, verwendbaren organischen Lösungsmitteln gehören Alkohole mit 1 bis 4 C-Atomen, insbesondere Methanol, Ethanol, Isopropanol und tert.-Butanol, Diole mit 2 bis 4 C-Atomen, insbesondere Ethylenglykol und Propylenglykol, sowie deren Gemische und die aus den genannten Verbindungsklassen ableitbaren Ether. Derartige wassermischbare Lösungsmittel sind in den erfindungsgemäßen Waschmitteln vorzugsweise in Mengen von nicht über 30 Gew.-%, insbesondere von 6 Gew.-% bis 20 Gew.-%, vorhanden.

Zur Einstellung eines gewünschten, sich durch die Mischung der übrigen Komponenten nicht von selbst ergebenden pH-Werts können die erfindungsgemäßen Mittel system- und umweltverträgliche Säuren, insbesondere Citronensäure, Essigsäure, Weinsäure, Äpfelsäure, Milchsäure, Glykolsäure, Bernsteinsäure, Glutarsäure und/oder Adipinsäure, aber auch Mineralsäuren, insbesondere Schwefelsäure, oder Basen, insbesondere Ammonium- oder Alkalihydroxide, enthalten. Derartige pH-Regulatoren sind in den erfindungsgemäßen Mitteln vorzugsweise nicht über 20 Gew.-%, insbesondere von 1,2 Gew.-% bis 17 Gew.-%, enthalten.

Die Herstellung fester erfindungsgemäßer Mittel bereitet keine Schwierigkeiten und kann in im Prinzip bekannter Weise, zum Beispiel durch Sprühtrocknen oder Granulation, erfolgen, wobei Persauerstoffverbindung und Bleichkatalysator gegebenenfalls später zugesetzt werden. Zur Herstellung erfindungsgemäßer Mittel mit erhöhtem Schüttgewicht, insbesondere im Bereich von 650 g/l bis 950 g/l, ist das aus der europäischen Patentschrift bekannte, einen Extrusionsschritt aufweisende Verfahren bevorzugt.

Bei erfindungsgemäßen Körperpflegemitteln handelt es sich in einer ersten bevorzugten Ausführungsform um ein Dauerwellmittel, insbesondere um eine Wellotion. Unter einer Wellotion wird dabei die Zubereitung verstanden, die das Reduktionsmittel enthält. Als Reduktionsmittel werden üblicherweise Mercaptoverbindungen und/oder Salze der schwefligen Säure eingesetzt. Bevorzugte Mercaptoverbindungen sind Thioglykolsäure, ihre physiologisch verträglichen Salze sowie ihre Ester. Weiterhin bevorzugt verwendet werden, wenn auch in geringerem Umfang, Cysteamin, Cystein, Thiomilchsäure, Thioäpfelsäure, Bunte Salze und α-Mercaptoethansulfonsäure.

Zur Überdeckung des Eigengeruchs dieser Mercaptoverbindungen enthalten die Wellotionen üblicherweise Parfümöle. Es besteht jedoch das Problem, daß Reste der Mercaptoverbindungen auch nach dem Fixieren der Dauerwelle am Haar verbleiben, in vielen Fällen sogar deutlich länger als die eingesetzten Parfümöle. Werden diese Haare später mit einem nur gering parfümierten Mittel, zum Beispiel einem Shampoo im Rahmen der üblichen Reinigung, behandelt, so lösen sich diese Reste der Mercaptoverbindung unter entsprechender Duftentfaltung sukzessive vom Haar. Diesem Problem kann durch erfindungsgemäße Verwendung der Alkylpolyglykoside in den Wellotionen in hervorragender Weise vorgebeugt werden. Die erfindungsgemäßen Wellotionen können des weiteren alle dem Fachmann bekannten Inhaltsstoffe, wie beispielsweise anionische Tenside, zwitterionische Tenside, ampholytische Tenside, nichtonische Tenside, kationische Tenside, Proteinhydrolysate, Verdickungsmittel, Strukturanten, kationische, anionische, zwitterionische, amphotere und nichtionische Polymere, Lösungsvermittler, Substanzen zur Einstellung des pH-Wertes, Wirkstoffe wie Allantoin, Pyrrolidoncarbonsäuren und Pflanzenextrakte, Lichtschutzmittel, Komplexbildner, Quell- und Penetrationsstoffe, Trübungsmittel, Farbstoffe, Parfümöle, Perlglanzmittel und Treibmittel. Diese Ausführungen bezüglich Dauerwellmitteln gelten in gleicher Weise auch für Depilatorien.

Gemäß einer weiteren bevorzugten Ausführungsform werden die Alkylpolyglykoside erfindungsgemäß in solchen Mitteln eingesetzt, die Amine enthalten. Auch hier erleichtern sie die Überdeckung unerwünschter Duftnuancen, die auf diese Amine zurückgehen. Solche Mittel sind, neben den bereits oben genannten Dauerwellmitteln beispielsweise Haarfärbemittel.

Gemäß einer dritten bevorzugten Ausführungsform kann die erfindungsgemäße Lehre dazu eingesetzt werden, den Parfümanteil in Körperpflegemitteln signifikant herabzusetzen. Dadurch ist es möglich, parfümierte Produkte auch für solche besonders empfindlichen Konsumenten anzubieten, die normal parfümierte Produkte aufgrund spezieller Unverträglichkeiten und Irritationen nur eingeschränkt oder überhaupt nicht verwenden können. In diesem Zusammenhang sind vor allem Hautpflegeprodukte und Deodoratien zu nennen.

### Beispiele

### Beispiel 1

Zum Nachweis der verstärkten Duftfixierung durch Alkylglykoside wurden vorgereinigte Läppchen (2 x 10 cm, Gewicht: 0,5 g) aus Baumwolle mit 1 g einer wäßrigen Lösung, enthaltend 12 Gew.-% C_{12/16}-Alkylglykosid (Oligomerisationsgrad 1,4) und 1 Gew.-% 3,3,5-Trimethylcyclohexylether 5 Minuten bei Raumtemperatur behandelt. Anschließend wurden die Läppchen 1,5 Minuten mit Wasser (38 °C, 1 Liter pro Minute) ausgewaschen und nach 30 Minuten Trocknen an der Luft einer GC-Headspaceanalyse unterzogen.

Zum Vergleich wurde eine wäßrige Lösung, die statt des Alkylglykosids die gleiche Menge Na-C_{12/14}-Alkylethersulfat (Ethoxylierungsgrad: 2) enthielt, getestet.

Auf den mit der Alkylglykosid-haltigen Mischung behandelten Läppchen wurde eine Duftstoffmenge nachgewiesen, die dreimal höher war als bei den Läppchen, die mit der Vergleichslösung behandelt worden waren.

### Beispiel 2

Zum Nachweis der Verstärkung der Reinigungsleistung von Alkylglykosid durch den Duftstoff wurde ein parfümfreies Vergleichswaschmittel (**V1**), enthaltend 19 Gew.-% Na-Alkylbenzolsulfonat, 2 Gew.-% 7-fach ethoxlierten Fettalkohol, 2 Gew.-% C₁₂₋₁₆-Alkyl-glucosid (Oligomerisierungsgrad ca. 1,4), 1 Gew.-% Talgalkylsulfat, 2,5 Gew.-% Seife, 27 Gew.-% Zeolith Na-A, 5 Gew.-% polymeres Polycarboxylat, 6 Gew.-% Natriumcarbonat, 3 Gew.-% Natriumsilikat, 25 Gew.-% Natriumsulfat, 1 Gew.-% Enzymgranulat (Protease, Cellulase), Rest auf 100 Gew.-% Wasser und Salze, mit ansonsten gleich zusammengesetzten Mitteln (**M1** bis **M4**), die aber die in der nachfolgenden Tabelle 1 angegebenen Zusätze von Duftstoffen (Ausgleich über Wasser) der in den Tabellen 2 bis 5 angegebenen Zusammensetzung enthielten, in einer Waschmaschine Miele® W 717 (1-Laugenprogramm Pflegeleicht/Fein, 30°C, Wasser mit 10°dH, Waschmitteldosierung 68 g, 2,5 kg sauberes Füllgewebe) in der Wirkung auf standardisierte Anschmutzungen (Tabelle 6) verglichen.

**Tabelle 1**

| Waschmittel + Duftstoff | | |
|---|---|---|
| Mittel | Duftstoff | Menge [Gew.-%] |
| **M1** | **D1** | 1 |
| **M2** | **D2** | 1 |
| **M3** | **D3** | 1 |
| **M4** | **D4** | 1 |

**Tabelle 2**

| Zusammensetzung von D1 | |
|---|---|
| **D1** | |
| Bergamotteöl | 50 Gew.-% |
| Dihydromyrcenol | 50 Gew.-% |

**Tabelle 3**

| Zusammensetzung von D2 | |
|---|---|
| **D2** | |
| Lilial | 15,0 Gew.-% |
| Lyral | 20,0 Gew.-% |
| Citronellol | 10,0 Gew.-% |
| Phenylethylalkohol | 10,0 Gew.-% |
| α-Hexylzimtaldehyd | 10,0 Gew.-% |
| Geraniol | 5,0 Gew.-% |
| Berzylaceton | 3,0 Gew.-% |
| Cyclamenaldehyd | 2,0 Gew.-% |
| Linalool | 2,0 Gew.-% |
| Boisambrene Forte | 1,7 Gew.-% |
| Ambroxan | 0,2 Gew.-% |
| Indol | 0,1 Gew.-% |
| Hedione | 16,0 Gew.-% |
| Sandelice | 5,0 Gew.-% |

**Tabelle 4**

| Zusammensetzung von D3 | |
|---|---|
| **D3** | |
| Bergamotteöl | 15,0 Gew.-% |
| Dihydromyrcenol | 20,0 Gew.-% |
| Citronenöl Messina | 7,5 Gew.-% |
| Mandarinenöl | 2,5 Gew.-% |
| Orangenöl süß | 5,0 Gew.-% |
| Allylamylglycolat | 2,0 Gew.-% |
| Cyclovertal | 0,5 Gew.-% |
| Lavandinöl grosso | 2,5 Gew.-% |
| Muskateller Salbeiöl | 1,0 Gew.-% |
| Lilial | 2,0 Gew.-% |
| ß-Damascone | 0,1 Gew.-% |
| Geraniumöl Bourbon | 3,0 Gew.-% |
| Hedione | 5,0 Gew.-% |
| Cyclohexylsalicylat | 4,0 Gew.-% |
| Vertofix Coeur | 10,0 Gew.-% |
| Iso-E-Super | 5,0 Gew.-% |
| Ambroxan | 1,6 Gew.-% |
| Fixolide NP | 10,0 Gew.-% |
| Evernyl | 1,0 Gew.-% |
| Dipropylenglycol | 2,3 Gew.-% |

**Tabelle 5**

| Zusammensetzung von D4 | |
|---|---|
| **D4** | |
| Phenylethylalkohol | 52,0 Gew.-% |
| DMBCA | 2,5 Gew.-% |
| Iraldein gamma | 5,0 Gew.-% |
| Phenylessigsäure | 0,5 Gew.-% |
| Geranylacetat | 2,0 Gew.-% |
| Benzylacetat | 3,0 Gew.-% |
| Rosenoxid L 10 % in DPG | 2,5 Gew.-% |
| Romillat | 2,0 Gew.-% |
| Irotyl | 0,5 Gew.-% |
| Cyclohexylsalicylat | 20,0 Gew.-% |
| Floramat | 10,0 Gew.-% |

**Tabelle 6**

| Waschergebnisse (Farbabstand ΔdE) | | | | | |
|---|---|---|---|---|---|
| Anschmutzung | Wert bei Mittel | | | | |
| | **V1** | **M1** | **M2** | **M3** | **M4** |
| Blut/Milch/Ruß | 24,6 | 25,1 | 26,2 | 24,9 | 26,7 |
| Curryketchup | nb | 40,5 | 41,1 | 41,6 | 40,7 |
| Ei/Ruß | 26,0 | 25,8 | 28,0 | 28,4 | 29,2 |
| Haferflocken/Kakao | 24,1 | nb | 28,6 | 28,9 | 28,2 |
| Kaffee | 17,6 | 19,5 | 19,0 | 18,1 | nb |
| Schuhcreme, schwarz | 30,9 | 33,0 | 31,0 | 31,0 | 32,5 |
| Johannisbeere | 12,8 | 16,9 | 15,1 | 14,2 | 13,2 |
| Öl | 30,4 | 32,9 | 30,6 | nb | 32,4 |
| nb: nicht bestimmt | | | | | |

## Patentansprüche

1. Verwendung von Alkylpolyglykosiden der allgemeinen Formel I
R¹-O(G)ₙ (I)
in der R¹ einen Alkylrest mit 8 bis 22 C-Atomen, G eine Glykoseeinheit und n eine Zahl von 1 bis 10 bedeuten, zur Fixierung von Duftstoffen an harten und/oder weichen Oberflächen.

2. Verwendung von Alkylpolyglykosiden der allgemeinen Formel I
R¹-O(G)ₙ (I)
in der R¹ einen Alkylrest mit 8 bis 22 C-Atomen, G eine Glykoseeinheit und n eine Zahl von 1 bis 10 bedeuten, zur Fixierung von Duftstoffen an menschlicher Haut, Haar und/oder Textilien.

3. Verwendung von Duftstoffen zur Erhöhung der Wasch- und/oder Reinigungsleistung von Alkylpolyglykosiden der allgemeinen Formel I
R¹-O(G)ₙ (I)
in der R¹ einen Alkylrest mit 8 bis 22 C-Atomen, G eine Glykoseeinheit und n eine Zahl von 1 bis 10 bedeuten

4. Verwendung nach einem der Ansprüche 1 bis 3, dadurch gekennzeichnet, daß man das Alkylpolyglykosid in Gewichtsmengen, bezogen auf den Duftstoff, im Bereich von 20:1 bis 1:1, insbesondere von 5:1 bis 1:1, einsetzt.

5. Verfahren zur Fixierung von Duftstoffen an harten und/oder weichen Oberflächen, dadurch gekennzeichnet, daß man die Oberfläche mit dem Duftstoff und einem Alkylpolyglykosid der allgemeinen Formel I
R¹-O(G)ₙ (I)
in der R¹ einen Alkylrest mit 8 bis 22 C-Atomen, G eine Glykoseeinheit und n eine Zahl von 1 bis 10 bedeuten, in Gegenwart von Wasser über einen Zeitraum von 2 Minuten bis 90 Minuten bei einer Temperatur unter 95 °C behandelt.

6. Verfahren nach Anspruch 5, dadurch gekennzeichnet, daß die Behandlungsdauer im Bereich von 5 Minuten bis 60 Minuten, insbesondere von 15 Minuten bis 45 Minuten liegt.

7. Verfahren nach Anspruch 5 oder 6, dadurch gekennzeichnet, daß die Temperatur im Bereich von 20 °C bis 60 °C, insbesondere von 20 °C bis 40 °C liegt.

8. Verfahren nach einem der Ansprüche 5 bis 7, dadurch gekennzeichnet, daß während der gesamten Behandlungszeit die Temperatur im Bereich von 20 °C bis 40 °C liegt.

9. Verfahren nach einem der Ansprüche 5 bis 8, dadurch gekennzeichnet, daß die Konzentration des Alkylpolyglykosids der allgemeinen Formel I im Bereich von 0,05 g/l bis 5 g/l, insbesondere von 0,1 g/l bis 3 g/l liegt.

10. Verfahren nach einem der Ansprüche 5 bis 9, dadurch gekennzeichnet, daß die Konzentration an Duftstoff in der wäßrigen Behandlungsflotte im Bereich von 0,004 g/l bis 0,12 g/l, insbesondere von 0,02 g/l bis 0,04 g/l liegt.

11. Wasch- oder Reinigungsmittel, enthaltend einen Duftstoff, ausgewählt aus Riechstoffverbindungen vom Typ der Ester, Ether, Aldehyde, Ketone, Alkohole und Kohlenwasserstoffe sowie deren Mischungen, und ein Alkylpolyglykosid der allgemeinen Formel I
R¹-O(G)ₙ (I)
in der R¹ einen Alkylrest mit 8 bis 22 C-Atomen, G eine Glykoseeinheit und n eine Zahl von 1 bis 10 bedeuten.

12. Mittel nach Anspruch 11, dadurch gekennzeichnet, daß es Alkylglykoside in Mengen von 1 Gew.-% bis 50 Gew.-%, insbesondere 1 Gew.-% bis 10 Gew.-% enthält.

13. Mittel nach Anspruch 11 oder 12, dadurch gekennzeichnet, daß das Gewichtsverhältnis von Alkylglykosid zu Duftstoff 10:1 bis 1:2, insbesondere 2:1 bis 1:1 beträgt.

14. Mittel nach einem der Ansprüche 11 bis 13, dadurch gekennzeichnet, daß es Lipase, insbesondere in einer solchen Menge, daß es eine lipolytische Aktivität im Bereich von 2 LU bis 2000 LU pro Gramm aufweist, enthält.
